# FASCICULE DE BREVET EUROPEEN

(11) **EP 2 121 551 B1**
(45) Date de publication et mention de la délivrance du brevet: **04.08.2010**
(21) Numéro de dépôt: 08799845.6
(22) Date de dépôt: 14.03.2008
(51) Int. Cl.: C07C 29/76

(54) **PROCEDE DE VAPORISATION DE GLYCEROL**
GLYCERINVERDAMPFUNGSVERFAHREN
GLYCEROL VAPORIZATION METHOD

(30) Priorité: 19.03.2007 FR 0753896
(43) Date de publication de la demande: 25.11.2009
(73) Titulaire: ARKEMA FRANCE, 92700 Colombes (FR)
(72) Inventeur: DUBOIS, Jean-Luc, 69390 Millery (FR)
(74) Mandataire: Bonnel, Claudine
(86) Numéro de dépôt international: PCT/FR2008/050438
(87) Numéro de publication internationale: WO 2008/129208

(56) Documents cités:
- EP-A- 0 982 283
- DATABASE CAPLUS [Online] CHEMICAL ABSTRACTS SERVICE, COLUMBUS, OHIO, US; TUICHIEV, I. S. ET AL: "Mass transfer during sorption from viscous solutions in a fluidized adsorbent bed" XP002458186 extrait de STN Database accession no. 1970:416796 & MASSOOBMENNYE PROTSESSY KHIMICHESKOI TEKHNOLOGII , NO. 4, 119-20 CODEN: MPKTB7; ISSN: 0542-9803, 1969,
- DATABASE WPI Week 199824 Thomson Scientific, London, GB; AN 1998-270248 XP002458187 & RU 2 093 503 C1 (NEVSKAYA KOSMETIKA STOCK CO) 20 octobre 1997 (1997-10-20)

## Description

La présente invention a pour objet un procédé de vaporisation de solutions aqueuses de glycérol dans un lit fluidisé contenant un solide inerte, permettant d'éliminer simultanément les impuretés présentes dans ces solutions ou générées lors de l'évaporation.

Le glycérol est un produit chimique, le 1,2,3-propane triol, qui peut être obtenu soit par synthèse chimique à partir de propylène, soit comme co-produit formé lors de la méthanolyse des huiles végétales.

La méthanolyse des huiles végétales peut s'effectuer selon différents procédés, notamment en utilisant une catalyse homogène telle que la soude ou le méthylate de sodium en solution dans le méthanol, ou en utilisant une catalyse hétérogène. On pourra se reporter sur ce sujet à l'article de D. Ballerini et al. dans l'actualité chimique de nov-déc 2002.

La méthanolyse des huiles végétales conduit, d'une part à des esters méthyliques, d'autre part à du glycérol. Les esters méthyliques sont employés notamment comme carburants ou combustibles dans le gazole et le fioul domestique. Avec le développement des carburants d'origines renouvelables, et notamment des esters méthyliques d'huiles végétales (EMHV), la production de glycérol selon cette voie d'obtention augmente fortement, le glycérol représentant de l'ordre de 10 % du poids de l'huile transformée.

Le glycérol issu des huiles végétales est un produit naturel d'origine renouvelable qui est ainsi de plus en plus disponible. Dans le cadre actuel de nouveau concept de chimie verte, et plus généralement de développement durable, il devient de plus en plus intéressant de valoriser ce produit.

Cependant, les procédés de production des EMVH conduisent à du glycérol plus ou moins pur et plus ou moins dilué dans de l'eau. Généralement, ce sont ces solutions aqueuses de glycérol plus ou moins pures qui sont dénommées glycérine, selon la définition adoptée par "the Soap and Detergent Association" (Soaps and Detergents : A theoretical and Practical Review, Miami Beach Fla., Oct 12-14 1994, chapter 6 pp172-206. Ed: L Spitz, AOCS Press, Champaign). La glycérine brute a en général une composition de l'ordre de 88...% de glycérol, 9-10 % d'eau et 2-3 % d'impuretés. Notamment, elle peut contenir des impuretés telles que des sels basiques (par exemple du sodium ou du potassium), des composés organiques non glycérineux, du méthanol ou des résidus d'huiles végétales. Dans certaines applications du glycérol, la présence de ces impuretés est particulièrement préjudiciable pour les réactions mises en oeuvre ou pour la qualité des produits finis. Par exemple, dans le cas de la production d'acroléine, la présence de sels de sodium ou de potassium est préjudiciable pour la réaction catalytique de déshydratation du glycérol en acroléine, car ces sels sont capables d'empoisonner les sites acides des catalyseurs mis en oeuvre.

Par conséquent, les solutions aqueuses de glycérol brutes ou glycérine nécessitent généralement un traitement préalable avant utilisation, ou un traitement de purification pour envisager de nouvelles applications.

De plus, il est souvent nécessaire non seulement d'éliminer les impuretés indésirables pour l'application envisagée, mais aussi de concentrer la solution aqueuse, voire vaporiser la solution aqueuse, certains procédés industriels mettant en oeuvre le glycérol sous forme vapeur. Ces opérations sont délicates car il est connu que le glycérol peut se décomposer, en particulier en acroléine, ou conduire à des polymères tels que du polyglycérol.

Différentes technologies de purification du glycérol ont été décrites dans la littérature. En effet, il s'agit d'un produit qui connaît plus de 1500 applications différentes, nécessitant toutes des qualités particulières, en particulier il existe un grade 'Pharmacopée' qui nécessite une haute pureté du glycérol.

Parmi les méthodes utilisées ou étudiées pour la purification et l'évaporation du glycérol, on citera notamment celles qui sont décrites par G.B. D'Souza, dans J. Am. Oil Chemists' Soc. November 1979 (Vol 56) 812A, par Steinberner U et al., dans Fat. Sci. Technol. (1987), 89 Jahrgang Nr.8, pp 297-303, et par Anderson D.D. et al. dans Soaps and Detergents : A theoretical and Practical Review, Miami Beach Fla., Oct 12-14 1994, chapter 6 pp172-206. Ed: L Spitz, AOCS Press, Champaign.

Les traitements de solutions brutes de glycérol proposés visent l'élimination des sels dissous et des impuretés organiques issues des corps gras, l'élimination de la couleur, une augmentation de la teneur en glycérol, ou la vaporisation du glycérol, selon l'application finale envisagée.

En particulier, pour atteindre ces objectifs, peuvent être réalisés une évaporation, une distillation, un traitement à la chaux (pour neutraliser les acides gras résiduels) suivi d'une filtration, un traitement par échange ionique ou par exclusion ionique, une séparation par osmose inverse ou une électrodialyse.

Des évaporateurs à multiples effets sont par exemple utilisés pour concentrer des solutions de glycérol diluées. Avec un évaporateur à triple effet, il est ainsi possible d'évaporer 2,4 kg d'eau avec 1 kg de vapeur.

La distillation est l'une des techniques utilisées pour concentrer et purifier de la glycérine. Comme le glycérol commence à se décomposer vers 202°C, soit bien en dessous de son point d'ébullition (293°C), il est nécessaire de distiller en plusieurs étapes la glycérine en utilisant une pression réduite. Dans certains cas, la distillation est faite par opérations discontinues, jusqu'à ce que les sels et les composés non volatils se soient accumulés suffisamment dans la capacité. L'opération est alors arrêtée et la capacité déchargée des impuretés avant de redémarrer la distillation. L'évaporation est effectuée sous vide, et la condensation partielle du glycérol (qui va condenser avant l'eau) en sortie de l'unité permet d'obtenir directement un glycérol concentré. Typiquement, des pressions de 10 mm de Hg sont utilisées, pour une température de 160-165°C, ce qui donne de faibles pressions partielles en glycérol en phase vapeur.

La glycérine distillée contient encore des composés colorés. Il est parfois nécessaire de décolorer la glycérine pour des applications pharmaceutiques et alimentaires. Typiquement du charbon actif est ajouté à la glycérine pour la décolorer.

La purification de la glycérine par exclusion ionique a aussi été développée et utilise des résines ioniques pour séparer les sels ioniques solubles en solution aqueuse des composés non ioniques comme le glycérol. C'est une technique qui évite la consommation de chaleur et de régénérants chimiques, et qui permet de purifier des flux fortement contaminés comme la glycérine brute, en utilisant seulement l'eau comme régénérant chimique.

Les solutions aqueuses de glycérol qui sont faiblement contaminées par des sels peuvent être échangées simplement sur des résines acides et basiques. Les solutions de glycérol ainsi purifiées peuvent alors être concentrées par évaporation.

La technique d'osmose inverse, basée sur une séparation sur membrane semi-perméable en appliquant une pression a été proposée pour la concentration de flux particulièrement dilués en glycérol.

Des solutions de glycérine et de soude dans du méthanol obtenues après transestérification de l'huile de colza ont été déminéralisées par électrodialyse membranaire pour produire de la glycérine pure. Cette technique est décrite dans la référence : Schaffner, F. et al. Proc. - World Filtr. Congr. 7th, Volume 2, 629-633.

Dans les méthodes proposées pour évaporer les solutions aqueuses de glycérol, le contrôle de la température est très critique car certaines réactions indésirables peuvent avoir lieu, telles que la formation de composés azotés par dégradation de matière protéinique présente dans la glycérine, la formation d'ester de glycérine volatil par réaction avec des savons de basse masse molaire, la formation de polyglycérol, la formation d'acroléine qui contribue aux odeurs du produit final. Il est donc important de limiter le temps de séjour de la glycérine à haute température, ainsi que cette température. Les procédés d'évaporation utilisés classiquement ne permettent donc pas d'avoir des pressions partielles élevées de glycérol en phase vapeur. Par ailleurs, il est souvent nécessaire de combiner plusieurs traitements pour obtenir le glycérol avec une pureté et à une concentration adaptées à l'application envisagée.

TUICHIEV, I. S. ET AL (Massoobmennye Protsessy Khimicheskoi Tekhnologii , 1969, no. 4, 119-120) décrit un procédé de purification du glycérol dans un lit fluidisé contenant une résine échangeuse d'ions.

La Société déposante a maintenant découvert de façon surprenante un procédé en une seule étape permettant de vaporiser une solution aqueuse de glycérol et d'éliminer simultanément les impuretés présentes ou générées au cours de l'évaporation dans cette solution.

La présente invention a donc pour objet un procédé de vaporisation de solutions aqueuses de glycérol (ou glycérine) dans un lit fluidisé contenant un solide inerte.

Selon le procédé de l'invention, la solution aqueuse est injectée directement dans un lit fluidisé contenant un solide inerte maintenu à une température suffisante pour permettre la vaporisation instantanée du glycérol et de l'eau.

Comme solides inertes, on peut utiliser par exemple du sable, de la poudre de verre ou de quartz, du carbure de silicium, ou des solides de faible surface spécifique, les solides de faible surface spécifique étant par nature réputés inertes, ils peuvent être composés d'alumine, silice ou silice-alumine. On ne sortirait pas du cadre de l'invention en utilisant comme solide inerte un sel minéral, tel que du chlorure de sodium (NaCl), du chlorure de potassium (KCI), du sulfate de sodium (Na₂SO₄) ou du sulfate de potassium (K₂SO₄). De préférence, on choisit le solide inerte parmi le sable, la silice, le quartz, le carbure de silicium.

La fluidisation peut être assurée par la vaporisation de la solution de glycérol, et/ou par un courant de gaz inerte (azote, CO₂, gaz de recycle...), ou d'air, d'oxygène, ou d'un mélange de gaz.

La température du lit fluidisé est généralement comprise entre 220 et 350°C, de préférence 260 à 320°C.

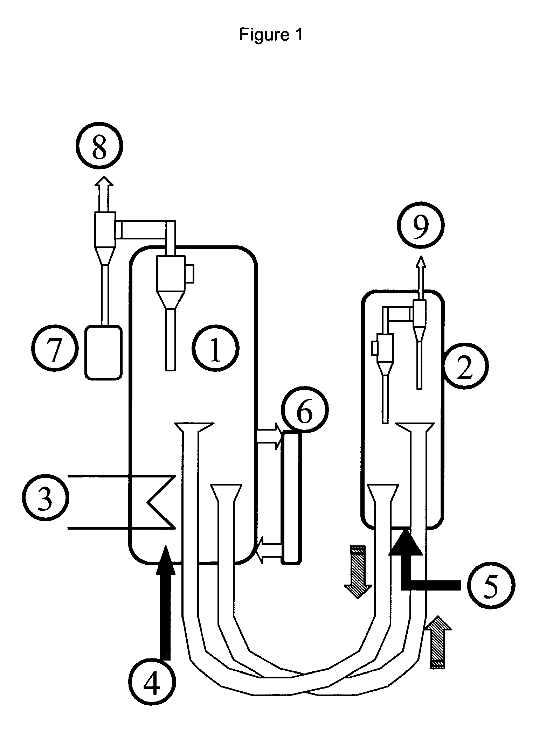

D'autres caractéristiques et avantages de l'invention ressortiront mieux à la lecture de la description qui suit et en référence à la figure unique annexée.

Le procédé selon l'invention conduit à des pressions partielles élevées de glycérol en phase vapeur, ce qui présente l'avantage de vaporiser le glycérol avec une productivité nettement plus élevée que celle obtenue avec une distillation sous vide.

Dans le procédé selon l'invention, les impuretés présentes dans la solution aqueuse sont éliminées simultanément, car la technique du lit fluidisé permet de soutirer en continu une partie du solide pour le régénérer ex-situ. Ainsi, les composés organiques présents dans la solution de glycérol, mais aussi les produits issus de la décomposition du glycérol lors de cette étape d'évaporation peuvent conduire à la formation de coke qui se dépose sur le solide inerte. Lorsque la solution aqueuse de glycérol contient des sels (par exemple chlorure de sodium, ou sulfate de sodium), ces sels se déposent aussi sur le solide inerte au cours de l'évaporation de la solution aqueuse de glycérol. Le solide inerte comportant le coke et/ou les sels minéraux peut alors être soutiré en continu pour être régénéré dans un autre réacteur, avant d'être renvoyé dans le lit fluidisé. L'élimination des sels minéraux peut s'effectuer par simple lavage à l'eau du solide, ou toute autre technique appropriée. La régénération du solide consiste en une combustion des dépôts solides, elle est généralement effectuée avec de l'air dans un réacteur qui peut être par exemple un autre lit fluidisé opéré en continu, un lit fixe, ou tout autre réacteur pouvant convenir. De préférence on utilisera un lit fluidisé opéré en continu. La combustion des dépôts carbonés sur le solide inerte permet non seulement de le régénérer mais aussi de le réchauffer avant de le retourner dans le lit fluidisé d'évaporation du glycérol. Cette combustion peut être effectuée en présence d'un combustible, par exemple du méthane, ce qui contribue à ramener le solide inerte à la température nécessaire à l'évaporation de la solution aqueuse de glycérol.

Par ailleurs, dans un lit fluidisé, les particules sont en mouvement les unes par rapport aux autres ce qui provoque une attrition du solide. Dans les lits fluidisés conventionnels, on cherche à limiter cette attrition qui consomme du solide et produit des fines particules. Dans le procédé selon l'invention, l'attrition permet d'éliminer une partie des dépôts qui se forment sur le solide inerte. Les fines particules ainsi formées par attrition sont éliminées en aval, par exemple par séparation dans un cyclone ou par filtration.

Dans un mode de réalisation du procédé selon l'invention, représenté sur la figure unique, une solution aqueuse de glycérol ou de la glycérine (4) est introduite dans un réacteur (1) contenant un lit fluidisé d'un solide inerte. La fluidisation peut éventuellement être assurée par un courant de gaz inerte (azote, CO₂, gaz de recycle...), ou d'air, d'oxygène, ou d'un mélange de gaz pouvant être choisis de manière à ce que la composition en 8 corresponde au mieux à l'alimentation des procédés en aval. Le lit fluidisé est chauffé par l'intermédiaire de l'échangeur de chaleur (3). Les vapeurs de glycérol et d'eau sont extraites du réacteur en (8) et une unité (7) permet de récupérer les fines particules de l'installation. Une unité (6) permet de laver le solide utilisé dans le lit fluidisé pour éliminer les sels minéraux déposés. Le réacteur (2) est un régénérateur du solide inerte dans lequel le solide soutiré de (1) est soumis à une combustion en présence de gaz de régénération (5) contenant de l'oxygène moléculaire et/ou des combustibles, le solide régénéré étant renvoyé dans le réacteur (1). Les gaz issus de l'unité de régénération sont évacués en (9).

Les vapeurs de glycérol obtenues selon ce procédé sont alors directement utilisables dans un procédé en aval mettant en oeuvre du glycérol sous forme gaz, tel que par exemple les procédés de production d'acroléine ou d'acide acrylique décrits dans les documents WO 06/087083, WO 06/087084, WO 06/114506, WO 07/090990 et WO 07/090991, ou un procédé de production d'acrylonitrile tel que décrit dans la demande FR07.53293. Elles peuvent également être condensées permettant de produire des solutions aqueuses de glycérol concentrées et purifiées.

L'invention porte aussi sur l'utilisation d'un lit fluidisé contenant un solide inerte pour vaporiser et purifier des solutions aqueuses de glycérol.

La présente invention va être maintenant décrite dans les exemples ci-après, de tels exemples étant donnés à but uniquement illustratif, et bien évidemment non limitatif.

### Exemples

On a mis 150 g de silice de granulométrie 100µm dans un lit fluidisé. Le lit fluidisé consiste en un tube d'acier inoxydable de 41 mm de diamètre et d'une hauteur totale de 790 mm. Le lit fluidisé est immergé dans un bain de sable fluidisé, chauffé par des éléments électriques installés à l'intérieur du bain. Trois thermocouples ont enregistré le gradient de température le long du tube. On a alimenté de l'air à un débit de 500 ml/min (conditions normales), en dessous d'une plaque métallique poreuse qui distribue le gaz à travers le diamètre du réacteur. Le mélange solution à tester/azote est alimenté par un tube métallique de 0,6 mm qui va jusqu'au fond du lit avec un débit massique de 0,5 g/min, en maintenant le débit d'azote à 1000 ml/min. La pression totale dans le lit fluidisé est de 1,2 bars et la température est maintenue à 310°C.

L'expérience a été réalisée avec une solution aqueuse contenant 18 % en poids de glycérol pur (99,5% Laboratoire MAT) et 2 % en poids de sel NaCl pendant 60 min, ce qui correspond à une masse totale de 0,6 g de sel alimentée à l'entrée du lit fluidisé.

Les produits sont collectés en sortie de lit fluidisé et condensés pour être analysés par conductivité.

La mesure de conductivité est réalisée sur un appareillage de type pH mètre/conductimètre Accumet Research AR-20. Les résultats sont rassemblés dans le tableau ci-après.

| Solution | Conductivité microS/cm |
|---|---|
| Glycérol pur | 9 |
| Eau distillée | 5 |
| Solution aqueuse à 18 % de glycérol pur et 2 % de sel | 4650 |
| Solution collectée dans le condenseur en sortie du lit fluidisé | 15 |

La masse totale de sel récupérée dans le condenseur est 0,0004 g, ce qui correspond à une efficacité de 99,9% pour la séparation du sel sur le lit fluidisé, traduite par la faible conductivité de la solution collectée dans le condenseur.

## Revendications

1. Procédé de vaporisation de solutions aqueuses de glycérol dans un lit fluidisé contenant un solide inerte maintenu à une température comprise entre 220 et 350°C.

2. Procédé selon la revendication 1, **caractérisé en ce que** le solide inerte est du sable, de la poudre de verre ou de quartz, du carbure de silicium, ou un solide de faible surface spécifique.

3. Procédé selon la revendication 1 ou 2, **caractérisé en ce que** le solide est régénéré dans un second lit fluidisé opéré en continu.

4. Utilisation d'un lit fluidisé contenant un solide inerte pour vaporiser et purifier des solutions aqueuses de glycérol.

## Claims

1. A method of vaporizing aqueous solutions of glycerol in a fluidized bed containing an inert solid maintained at a temperature between 220 and 350°C.

2. The method as claimed in claim 1, **characterized in that** the inert solid is sand, glass or quartz powder, silicon carbide, or a solid having a low specific surface area.

3. The method as claimed in claim 1 or 2, **characterized in that** the solid is regenerated in a second continuously operated fluidized bed.

4. The use of a fluidized bed containing an inert solid for vaporizing and purifying aqueous solutions of glycerol.

## Patentansprüche

1. Verfahren, bei dem wässerige Lösungen von Glycerol in einer Wirbelschicht verdampft werden, die einen inerten Feststoff enthält und auf einer Temperatur im Bereich von 220 bis 350 °C gehalten wird.

2. Verfahren nach Anspruch 1, **dadurch gekennzeichnet, dass** es sich bei dem inerten Feststoff um Stand, Glaspulver oder Quarzpulver, Siliciumcarbid oder um eignen Feststoff mit kleiner spezifischer Oberfläche handelt.

3. Verfahren mach Anspruch 1 oder 2, **dadurch gekennzeichnet, dass** der Feststoff in einer zweiten Wirbelschicht regeneriert wird, die kontinuierlich betrieben wird.

4. Verwendung einer Wirbelschicht, die einen inerten feststoff enthält, zum Verdampfern und zum Reinigen von wässerigen Lösungen von Glycerol.
